# EUROPÄISCHE PATENTSCHRIFT

(11) **EP 3 770 144 B1**
(45) Veröffentlichungstag und Bekanntmachung des Hinweises auf die Patenterteilung: **06.09.2023**
(21) Anmeldenummer: 19188145.7
(22) Anmeldetag: 24.07.2019
(51) Int. Cl.: C07C 45/50, C07C 47/02

(54) **KONTINUIERLICHES HYDROFORMYLIERUNGSVERFAHREN MIT KATALYSATORSUBSTITUTION**
CONTINUOUS HYDROFORMYLATION PROCESS WITH CATALYST SUBSTITUTION
PROCÉDÉ D'HYDROFORMYLATION CONTINUE À SUBSTITUTION DE CATALYSEUR

(43) Veröffentlichungstag der Anmeldung: 27.01.2021
(73) Patentinhaber: OQ Chemicals GmbH, 40789 Monheim am Rhein (DE)
(72) Erfinder: Hensel, Alexander, 45130 Essen (DE); Fernandez Diaz, Ruben, 46539 Dinslaken (DE); Greb, Wolfgang, 46535 Dinslaken (DE); Botterhuis, Jörg, 45570 Marl (DE); Meier, Gregor, 47057 Duisburg (DE)
(74) Vertreter: Michalski Hüttermann & Partner Patentanwälte mbB

(56) Entgegenhaltungen:
- EP-A1- 0 269 011
- WO-A1-2013/153136

## Beschreibung

Die vorliegende Erfindung betrifft ein kontinuierliches Zwei-Phasen Hydroformylierungsverfahren zur Darstellung von Aldehyden aus Olefinen mittels Kohlenmonoxid, Wasserstoff und einem Übergangsmetallkatalysator in einer Reaktionszone, wobei das Übergangsmetall in Form eines wasserlöslichen Katalysatorkomplexes vorliegt, wobei das Verfahren ein oder mehrmals die folgenden Schritte umfasst:
a) Hydroformylieren durch zur Reaktion bringen der Olefine, Kohlenmonoxid und Wasserstoff an einem wasserlöslichen Übergangsmetallkatalysator aufweisend wasserlösliche Organophosphorliganden in der Reaktionszone;
b) Verringerung der Konzentration der Olefine in der Reaktionszone durch Reduzierung der Olefin-Zufuhr zur Reaktionszone um größer oder gleich 50% und kleiner oder gleich 100% bezogen auf die Olefin-Konzentration in der Reaktionszone im Verfahrensschritt a) und Entnehmen mindestens eines Teils der Katalysatorlösung aus dem Reaktionssystem, wobei die Teilschritte der Katalysatorlösung-Entnahme und der Verringerung der Olefin-Konzentration in dieser oder umgekehrter Reihenfolge, gleichzeitig oder nacheinander erfolgen können;
c) Zuführen eines Lösemittels, einer Übergangsmetallquelle und wasserlöslicher Organophosphorliganden zum Reaktionssystem, wobei die Zuführung der Komponenten gleichzeitig oder in beliebiger Reihenfolge nacheinander erfolgen kann;
d) Erhöhung der Konzentration der Olefine in der Reaktionszone durch Erhöhung der Olefin-Zufuhr zur Reaktionszone und Hydroformylieren durch zur Reaktion bringen der Olefine mit Kohlenmonoxid und Wasserstoff.

Hydroformylierungsverfahren sind seit langem in den unterschiedlichsten Verfahrensführungen bekannt. Allen gemein ist, dass Olefine in Gegenwart von Hydroformylierungskatalysatoren mit Wasserstoff und Kohlenmonoxid, letztere üblicherweise gemeinsam der Reaktion als Synthesegas zugeführt, zu Aldehyden umgesetzt werden. So ergibt beispielsweise die Hydroformylierung von Propylen ein Gemisch aus geradkettigem n- und verzweigtem i-Butyraldehyd, von denen das n-Isomer üblicherweise das wirtschaftlich gewünschte Produkt darstellt.

Während zu Beginn die Umsetzungen an Cobalt-Katalysatoren durchgeführt wurden, hat sich mittlerweile Rhodium als vorteilhaft durchgesetzt. Hohe Produktivitäten und bevorzugte n/i-Verhältnisse lassen sich, im Vergleich zur Cobalt-Katalyse, mittels RhodiumKomplexkatalysatoren unter vergleichsweise milderen Reaktionsbedingungen erhalten. Zudem können durch die Wahl geeigneter Liganden die Löslichkeitseigenschaften des Katalysators innerhalb gewisser Grenzen eingestellt werden, welches eine Wiedergewinnung des teuren Katalysators erleichtert und darüber hinaus die katalytischen Eigenschaften des Rhodium-Liganden-Komplexes beeinflusst.

Bedingt durch die Flexibilität der Umsetzung haben sich für Rhodium katalysierte Hydroformylierungen prinzipiell zwei unterschiedliche Reaktionsführungen herauskristallisiert. Beide unterscheiden sich in der Art der Abtrennung des Rohproduktes vom Katalysator und werden als Flüssig/Flüssig- oder Dampfverfahren bezeichnet.

In Flüssig/Flüssig-Verfahren erfolgt die Trennung von Rohprodukt und anderen Bestandteilen, wie beispielsweise dem Katalysator, durch Phasentrennung in eine flüssige organische sowie eine wässrige Phase. Beide Phasen können unabhängig voneinander abgezogen werden, wobei das Rohprodukt, ggf. mit Nebenprodukten, in der organischen und der Katalysator in der wässrigen Phase vorliegt. An dieser Aufarbeitung ist vorteilhaft, dass aufgrund der zwei-phasigen Ausgestaltung die Trennoperation für einen breiten Bereich unterschiedlicher Produktaldehyde durchgeführt werden kann, unabhängig von weiteren Eigenschaften, wie beispielsweise die Flüchtigkeit des Produkts und gegebenenfalls weiterer Nebenprodukte. Des Weiteren kann diese Aufarbeitung ohne große thermische Belastung durchgeführt werden.

Im Rahmen der zweiten Aufarbeitung, dem Dampfverfahren, erfolgt die Abtrennung des Rohprodukts in der Gasphase. Als Funktion des Siedepunktes der eingesetzten Olefine kann das Dampfverfahren dabei in zwei unterschiedlichen Ausgestaltungen erfolgen. Die aus leicht verdampfbaren Olefinen gewonnenen, niederen Aldehyde mit entsprechend niedrigem Siedepunkt werden üblicherweise zuerst kondensiert und als Flüssigkeit von den restlichen, gasförmigen Bestandteilen, beispielsweise nicht umgesetztem Olefin und Synthesegas, abgetrennt und dann einer thermischen Auftrennung unterzogen. Nicht umgesetzte Edukte werden gasförmig zum ursprünglichem oder zu weiteren Nachreaktoren zurückgeführt. Insofern spricht man von einem Gasrückführungs- oder Gaskreislaufverfahren. Höhere Olefine werden meistens mittels eines Flüssigrückführungsverfahrens umgesetzt, in denen ein Lösungsvolumen kontinuierlich aus der Reaktionszone abgezogen und einer ein- oder mehrstufigen Verdampfung zugeführt wird. Die anfallende abgetrennte Katalysatorlösung kann dann wieder in den Reaktionsort eingespeist werden.

Als Funktion der Olefin-Edukte, des apparativen Aufbaus und der gewählten Reaktionsbedingungen ergeben sich also vielfältige verfahrenstechnische Kombinationsmöglichkeiten, welche unterschiedliche Prozessparameter und Katalysatorsysteme erfordern. Sämtlichen Verfahrensführungen ist jedoch gemein, dass unter den Reaktionsbedingungen oder als Folge der Aufarbeitungsoperationen der eingesetzte Katalysator mit der Zeit altert. Dadurch sinken die Ausbeuten und/oder das erreichbare Isomerenverhältnis verschiebt sich ungünstig. Zwar lassen sich durch Anpassung der Reaktionsbedingungen, beispielsweise durch eine Erhöhung der Temperatur in der Reaktionszone, die Folgen der Alterung zum Teil kompensieren, letztendlich muss man aber zur Aufrechterhaltung einer wirtschaftlichen Reaktion innerhalb regelmäßiger Zeitabstände die Reaktion beenden und den verbrauchten Katalysator aufarbeiten.

In der Patentliteratur finden sich einige Vorschläge zu kontinuierlichen Verfahrensführungen, welche in langen Zeiträumen hohe Ausbeuten ermöglichen sollen.

So offenbart beispielsweise die EP 2 417 093 B1 ein Verfahren, bei dem das Abgas, das bei der Hydroformylierung ungesättigter Verbindungen in Gegenwart einer wässrigen, wasserlösliche Rhodiumkomplexverbindungen enthaltenden Katalysatorlösung anfällt (erste Reaktionsstufe), einem homogenen Reaktionssystem zugeführt wird, in dem die Restmengen der olefinisch ungesättigten Verbindungen aus der ersten Reaktionsstufe in Gegenwart von Komplexverbindungen des Rhodiums mit organischen Phosphor(III)-Verbindungen umgesetzt werden (zweite Reaktionsstufe). Der Austrag der zweiten Reaktionsstufe wird zunächst entgast und anschließend über zwei Entspannungsstufen unter Abgasbildung geführt. Die dabei anfallende flüssige Phase wird destilliert, wobei der erhaltene rhodiumhaltige Rückstand teilweise ausgeschleust und teilweise in die zweite Reaktionsstufe unter Zugabe von frischem Rhodium und frischen organischen Phosphor(III)-Verbindungen zurückgeführt wird. Darüber hinaus offenbart dieser Schritt, dass die erhaltene Aktivität nach Zusatz frischen Rhodiums geringer ist und dies vermutlich auf katalytisch inaktives Metall zurückzuführen ist.

Die EP 2 836 474 B1 offenbart ein Verfahren zur kontinuierlichen Hydroformylierung, bei dem ein Olefinausgangsmaterial, welches wenigstens ein Olefin mit 3 bis 20 Kohlenstoffatomen enthält, bei erhöhter Temperatur und bei erhöhtem Druck in einer Reaktionszone mit Synthesegas in Gegenwart eines mit einem Organophosphorliganden komplexierten, homogenen Übergangsmetall-Katalysators und freiem Liganden umgesetzt wird, wobei der Katalysator in situ in der Reaktionszone gebildet wird und man der Reaktionszone zum Ausgleich von Katalysatorverlusten eine Lösung einer Übergangsmetallquelle zufügt, dadurch gekennzeichnet, dass man kontinuierlich die Raum-Zeit-Ausbeute der Reaktionszone an Hydroformylierungsprodukt bestimmt und die Zugabegeschwindigkeit der Übergangsmetallquelle in die Reaktionszone in Abhängigkeit von der Raum-Zeit-Ausbeute steuert, wobei man
- ein Stellmittel zum Einstellen der Zugabegeschwindigkeit der Übergangsmetallquelle in die Reaktionszone vorsieht,
- einen Zielwert für die Raum-Zeit-Ausbeute der Reaktionszone an Hydroformylierungsprodukt festlegt,
- den Istwert für die Raum-Zeit-Ausbeute ermittelt,
- nach Erreichen eines unteren Grenzwerts für die Abweichung des Istwerts vom Zielwert die Menge an Übergangsmetallquelle ermittelt, die erforderlich ist, den Katalysatorverlust auszugleichen, und
- der Reaktionszone eine Lösung der Übergangsmetallquelle zufügt, wobei die Zugabegeschwindigkeit der Übergangsmetallquelle in die Reaktionszone so gesteuert wird, dass die Raum-Zeit-Ausbeute einen oberen Grenzwert für die Abweichung des Istwerts vom Zielwert nicht überschreitet.

Eine weitere Möglichkeit zur Erhöhung der Effizienz des Katalysatorsystems wird in der EP 0 246 475 A1 offenbart. Diese lehrt ein Verfahren zur Herstellung von Aldehyden durch Umsetzung ungesättigter Verbindungen mit Kohlenmonoxid und Wasserstoff bei Temperaturen von 20 bis 150°C und Drücken von 0,1 bis 20 MPa in flüssiger Phase in Gegenwart von Wasser und einer wasserlöslichen, Rhodium enthaltenden Komplexverbindung als Katalysator, wobei die Rhodium-Komplexverbindung vor Einsetzen der Hydroformylierungsreaktion aus dem in einem aliphatischen, cycloaliphatischen oder aromatischen Kohlenwasserstoff gelösten Rhodiumsalz einer Carbonsäure mit 2 bis 18 Kohlenstoffatomen durch Reaktion mit Kohlenmonoxid und Wasserstoff bei Drücken von 0,1 bis 1,8 MPa und Temperaturen von 50 bis 100°C vorgebildet wird, wobei die Präformierung in Gegenwart jener wässrigen Lösung eines wasserlöslichen Triarylphosphins erfolgt oder diese wässrige Lösung nach der Präformierung der zuvor hergestellten Rhodium-Komplexverbindung zugesetzt wird.

Aus der EP0269011 A1 ist ein Verfahren zur Herstellung von Aldehyden durch Umsetzung von Olefinen mit Kohlenmonoxid und Wasserstoff in Gegenwart wasserlöslicher Rhodiumkomplexkatalysatoren bekannt. In diesem verfahren wird zusammen mit dem Reaktionsprodukt ein Teil der wäßrigen Lösung des Katalysatorsystems abgezogen und durch frische Lösung ersetzt.

Die WO2013153136 A1 betrifft ein Verfahren zur Ergänzung des Katalysators bei der kontinuierlichen Hydroformylierung im laufenden Betrieb.

Es sind aus der Literatur also vielfältige Verfahrensführungen bekannt, in denen der Reaktionszone einer wie auch immer ausgestalteten, homogen ablaufenden Hydroformylierung weitere Katalysator-Teilmengen, entweder eines aufgearbeiteten zuvor aus der Reaktionszone entfernten, eines frischen oder eine Kombination beider Katalysatortypen, hinzugefügt werden. Für eine in Zwei-Phasen ablaufende Hydroformylierung, wie beispielsweise nach dem Ruhrchemie-/Rhone-Poulenc-Verfahren, war jedoch bislang keine entsprechende Verfahrensweise zur Entnahme und Zugabe einer Katalysatorteilmenge möglich. Somit besteht ein großes Interesse an kontinuierlichen großtechnischen Prozessführungen für Hydroformylierungen, welche in der Lage sind, über längere Zeiträume gleichbleibend hohe Selektivitäten und hohe Produktausbeuten zu liefern.

Es ist daher die Aufgabe der vorliegenden Erfindung, ein verbessertes kontinuierliches Hydroformylierungsverfahren bereitzustellen, welches den Nachteilen des Standes der Technik zumindest teilweise entgegenwirkt.

Zur Lösung dieser Aufgabe wird daher ein Verfahren nach Anspruch 1 vorgeschlagen. Bevorzugte Ausführungsformen des erfindungsgemäßen Verfahrens sind in den Unteransprüchen wiedergegeben.

Erfindungsgemäß gelöst wird die Aufgabe durch ein kontinuierliches Zwei-Phasen Hydroformylierungsverfahren zur Darstellung von Aldehyden aus Olefinen mittels Kohlenmonoxid, Wasserstoff und einem Übergangsmetallkatalysator in einer Reaktionszone, wobei das Übergangsmetall in Form eines wasserlöslichen Katalysatorkomplexes vorliegt, wobei das Verfahren ein oder mehrmals die folgenden Schritte umfasst:
a) Hydroformylieren durch zur Reaktion bringen der Olefine, Kohlenmonoxid und Wasserstoff an einem wasserlöslichen Übergangsmetallkatalysator aufweisend wasserlösliche Organophosphorliganden in der Reaktionszone;
b) Verringerung der Konzentration der Olefine in der Reaktionszone durch Reduzierung der Olefin-Zufuhr zur Reaktionszone um größer oder gleich 50% und kleiner oder gleich 100% bezogen auf die Olefin-Konzentration in der Reaktionszone im Verfahrensschritt a) und Entnehmen mindestens eines Teils der Katalysatorlösung aus dem Reaktionssystem, wobei die Teilschritte der Katalysatorlösung-Entnahme und der Verringerung der Olefin-Konzentration in dieser oder umgekehrter Reihenfolge, gleichzeitig oder nacheinander erfolgen können;
c) Zuführen eines Lösemittels, einer Übergangsmetallquelle und wasserlöslicher Organophosphorliganden zum Reaktionssystem, wobei die Zuführung der Komponenten gleichzeitig oder in beliebiger Reihenfolge nacheinander erfolgen kann;
d) Erhöhung der Konzentration der Olefine in der Reaktionszone durch Erhöhung der Olefin-Zufuhr zur Reaktionszone und Hydroformylieren durch zur Reaktion bringen der Olefine mit Kohlenmonoxid und Wasserstoff.

Überraschenderweise hat sich gezeigt, dass mittels oben angegebener Verfahrensschritte kontinuierliche Hydroformylierungen nach dem Ruhrchemie-/Rhone-Poulenc-Verfahren quasi unbegrenzt gefahren werden können, ohne dass sich die Notwendigkeit ergibt, aufgrund der Katalysatoralterung den gesamten Prozess zu beenden und den Katalysator in seiner Gesamtheit der Reaktionszone zu entziehen und aufarbeiten zu müssen. Es lassen sich mit einem sehr geringen Eingriff in den Hydrofromylierungsprozess als solchen, über lange Zeiträume hohe Produktivitäten und hohe Selektivitäten erreichen, wobei der Gesamtprozess im Vergleich zu den Standardverfahren aus dem Stand der Technik vorteilhafterweise auch mit weniger Katalysator- und Ligandeneinsatz gefahren werden kann. Es ist möglich, aktive Katalysatoren mit hohem Wirkungsgrad direkt in der Reaktionszone zu erhalten. Die aus Metall und Ligand gebildeten katalytisch aktiven Komplexe sind untereinander im Gleichgewicht, das in Abhängigkeit der chemischen Umgebung und der physikalischen Randbedingungen zu mehr aktiven sowie selektiven Komplexen verschoben werden kann.

Insofern erfolgt für das Zentralatom der Katalysatorkomplexe unter den Reaktionsbedingungen der Reaktionszone in der Regel ein Austausch der Liganden in der Ligandensphäre. Die Koordination der potentiellen Liganden am Metallzentrum bestimmt dabei die katalytische Aktivität und insbesondere auch die induzierte Regioselektivität und somit letztendlich das erhältliche Isomerenverhältnis im Rohprodukt. Die Ausbildung der Komplexe wird dabei neben den zum Aufbau des gewünschten Katalysators eingesetzten Liganden, d.h. Wasserstoff, Kohlenmonoxid, Olefin und Organophosphorligand, auch durch weitere, in der Reaktionszone vorliegende Abbau- und Nebenprodukte beeinflusst, welche durch ihren Einbau in die Ligandensphäre der Metallkomplexe zu ungewünschten Eigenschaften und zu einer Verschlechterung der Syntheseleistung beitragen können. Insofern ist es nicht vorhersehbar gewesen, dass eine Umwandlung in einen hocheffizienten Katalysator in einer solchen Umgebung der Reaktionszone gelingt. Ohne durch die Theorie gebunden zu sein, wird die Tatsache, dass ein hocheffizienter Katalysator erhalten wird, auf ein synergistisches Zusammenwirken mindestens zweier Ursachen zurückgeführt. Zum einen erfolgt die Zugabe neuen Katalysatormetalls in Abwesenheit des Olefins in der Reaktionszone und zum anderen scheint die um die Abbau- und Nebenprodukte verdünnte Katalysatorlösung in der Reaktionszone für die Ausbildung sehr selektiver und effizienter Katalysatorkomplexe förderlich. Diese Theorie wird dadurch gestützt, dass Supplemente des Katalysatormetalls unter Anwesenheit des Olefins und zu einer unverdünnten Lösung zu keiner nennenswerten Steigerung der Aktivität und keiner Verbesserung der n/i-Selektivität führen. Üblicherweise werden zur Verlängerung der Katalysator-Standzeiten in den hier betrachteten kontinuierlichen Hydroformylierungsprozessen nur weitere Organophosphorliganden zugeführt, welche im Rahmen einer Gleichgewichtsreaktion die in den Metallkomplexen vorliegenden Katalysatorgifte, wie beispielsweise LigandenAbbauprodukte, verdrängen und so den Katalysator wieder in eine aktivere Form überführen sollen. Letztere Erhaltungsmaßnahmen verlangen aber hohe Ligandenmengen und können schlussendlich einen Abbruch der kontinuierlichen Reaktion und einen Gesamtaustausch des Katalysators nicht verhindern, da durch die Zersetzung der vorliegenden Liganden die Konzentration der Katalysatorgifte kontinuierlich erhöht wird. Auch die durch Kreislaufverfahren zur Reaktionszone zurückgeführten Katalysatoren scheinen, trotz zusätzlicher Supplementierung frischen Katalysatormetalls, wahrscheinlich basierend auf einer anderen chemischen Umgebung und/oder aufgrund der doch unterschiedlichen Volumenströme, nicht zur Ausbildung ähnlich effizienter Katalysatorkomplexe geeignet.

Das erfindungsgemäße Verfahren betrifft ein kontinuierliches Zwei-Phasen Hydroformylierungsverfahren. In dem erfindungsgemäßen Verfahren liegen das aktive Katalysatormetall in einer wässrigen und die gebildeten organischen Aldehyde, zusammen mit gegebenenfalls gebildeten organischen Nebenprodukten, in einer aus ihnen gebildeten organischen Phase vor. Die Umsetzung der Olefine erfolgt in einem zwei-phasigen System aus organischer und wässriger Phase. Der aktive Metallkomplex ist wiederum in der wässrigen Phase homogen gelöst. Das Verfahren ist ein kontinuierliches Verfahren, da zur Durchführung der eigentlichen Umsetzung die Edukte kontinuierlich in die Reaktionszone eingespeist und die gebildeten Produkte kontinuierlich aus dieser entfernt werden.

Mittels des erfindungsgemäßen Verfahrens werden aus Olefinen Aldehyde dargestellt. Beispielhaft kann die Umsetzung anhand der Umsetzung von Propen dargestellt werden:

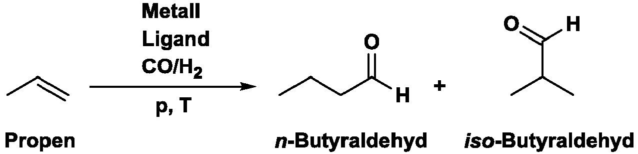

Aus der Hydroformylierung wird sowohl der geradkettige n- wie auch der verzweigte i-Aldehyd erhalten. Die einsetzbaren Olefine können linear oder verzweigt sein und eine endständige oder innenständige Doppelbindung aufweisen. Beispiele für solche Olefine sind: Ethylen, Propylen, 1- Buten, 2-Buten, 1-Penten, 2-Methyl-1-buten, 1-Hexen, 1-Hepten, 1-Octen, 4,4- Dimethyl-1-nonen, 1-Dodecen. Bevorzugt können lineare Olefine mit 2 bis 8 Kohlenstoffatomen wie Ethylen, Propylen, 1-Buten, 1-Penten, 1-Hexen, 1-Hepten und 1-Octen oder Mischungen mindestens zweier möglicher Doppelbindungsisomere daraus eingesetzt werden.

Die Umsetzung oder das zur Reaktion bringen der Olefine erfolgt in Gegenwart von Kohlenmonoxid und Wasserstoff. Üblicherweise erfolgt der Einsatz dieser Edukte gemeinsam als Synthesegas. Der Gesamtdruck von Wasserstoff und Kohlenmonoxid kann dabei 1 - 200 bar (100 - 2*10⁴ kPa), vorzugsweise 10 bis 100 bar (1*10³ bis 1*10⁴ kPa) betragen. Die Zusammensetzung des Synthesegases, d.h. das Verhältnis von Kohlenmonoxid zu Wasserstoff, kann in weiten Grenzen variiert werden. Im Allgemeinen setzt man Synthesegas ein, in dem das Volumenverhältnis von Kohlenmonoxid zu Wasserstoff 1:1 beträgt oder von diesem Wert nur wenig abweicht.

Zur Umsetzung wird ein Übergangsmetallkatalysator eingesetzt, wobei der Übergangsmetallkatalysator in Form eines wasserlöslichen Katalysatorkomplexes vorliegt. Einsetzbare Übergangsmetalle sind beispielsweise die Übergangsmetalle der 8.-10. Nebengruppe wie beispielsweise Cobalt, Rhodium, Iridium, Eisen, Ruthenium, Osmium und Platin. Bevorzugt kann Cobalt oder Rhodium, davon besonders bevorzugt Rhodium eingesetzt werden. Die Umsetzung und Katalyse erfolgt in der wässrigen Phase und erfordert die Wasserlöslichkeit der Metallkomplexe. Üblicherweise wird angenommen, dass der Katalysator in der wässrigen Phase sowohl mit den Edukten als Liganden als auch mit den wasserlöslichen Organophosphorliganden koordiniert vorliegt, wobei insbesondere letztere zur Wasserlöslichkeit des Komplexes beitragen. Im Laufe der Hydroformylierung und als Funktion der chemischen Umgebung kann sich aber die Ligandensphäre, beispielsweise durch Aufnahme von Wasserstoff, Olefin oder Kohlenmonoxid ändern, sodass angenommen wird, dass Katalysatorkomplexe mit gemischten und unterschiedlichen Ligandensphären vorliegen können, wobei diese Komplexe letztendlich immer noch wasserlöslich sind.

Das erfindungsgemäße Verfahren kann ein oder mehrmals die beanspruchten Schritte umfassen. Das Verfahren kann bevorzugter Weise häufiger durchgeführt werden, um die Katalysatorstandzeit und somit die gesamte Verfahrensdauer zu verlängern. So ist es beispielsweise möglich, das Verfahren als Funktion der aktuellen Syntheseleistung, des gemessenen Isomeren-Verhältnisses oder aber als Funktion der Salz-Fracht der wässrigen Katalysatorlösung durchzuführen. Das Verfahren kann beispielsweise 1-100 mal, bevorzugt 1-50 mal, des Weiteren bevorzugt 1-10 mal durchgeführt werden. Insbesondere ist erfindungsgemäß nicht vorgesehen, dass das Verfahren kontinuierlich, d.h. fortwährend im Rahmen einer Hydroformylierung durchgeführt wird. Das bedeutet, dass das Durchführen eines reinen Kreislaufverfahrens mit kontinuierlicher Abtrennung und/oder Aufbereitung des Katalysators nicht unter das erfindungsgemäße Verfahren zu fassen ist.

Im Verfahrensschritt a) erfolgt ein Hydroformylieren durch zur Reaktion bringen der Olefine mit Kohlenmonoxid und Wasserstoff an einem wasserlöslichen Übergangsmetallkatalysator aufweisend wasserlösliche Organophosphorliganden in der Reaktionszone. In diesem Verfahrensschritt erfolgt die katalytische Umsetzung der Edukt-Olefine zu den gewünschten Aldehyden. Die Umsetzung findet üblicherweise in einem Reaktor statt, in welchem die Edukte gasförmig eingespeist werden. Innerhalb des Reaktors liegt der Katalysator in einer flüssigen, wässrigen Phase gelöst vor, welche von den gasförmigen Edukten durchströmt und abgesättigt wird. Dieser Ort im Reaktor bildet erfindungsgemäß die Reaktionszone.

Unter dem Begriff wasserlösliche Organophosphorliganden können beispielsweise Verbindungen aus der Klasse der Triarylphosphine und der Diphosphine verstanden werden, die auf Grund des Vorliegens einer oder mehrerer Sulfonat- oder Carboxylatgruppen im Molekül in Wasser löslich sind. Die wasserlöslichen Triaryl-, insbesondere Triphenylphosphine folgen der allgemeinen Formel (I), 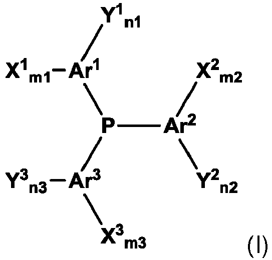 wobei Ar¹, Ar², Ar³ jeweils unabhängig voneinander eine Phenyl- oder Naphthylgruppe, Y¹, Y², Y³ jeweils unabhängig voneinander eine geradkettige oder verzweigte Alkylgruppe mit 1 bis 8 C-Atomen, eine geradkettige oder verzweigte Alkoxygruppe mit 1 bis 8 C-Atomen, ein Halogenatom, eine OH-, CN-, NO₂- oder NR¹R²-Gruppe, in der R¹ und R² jeweils für eine geradkettige oder verzweigte Alkylgruppe mit 1 bis 8 C-Atomen stehen, bedeuten, X¹, X², X³ unabhängig voneinander für einen Carboxylat- (COO⁻-) und/oder einen Sulfonat-(-SO₃⁻) Rest stehen, m₁, m₂, m₃ gleiche oder verschiedene Zahlen von 0 bis 3 sind, wobei mindestens eine Zahl m₁, m₂, m₃ gleich oder größer als 1 ist, und n₁, n₂, n₃ gleiche oder verschiedene ganze Zahlen von 0 bis 5 sind. Die negative Ladung von X¹, X², X³ ist durch Gegenionen, beispielsweise Alkalimetallionen, Erdalkalimetall- oder Zinkionen, Ammonium- oder quaternäre Ammoniumionen neutralisiert. Bevorzugt werden wasserlösliche Triarylphosphine der vorstehend beschriebenen allgemeinen Formel, in der Ar jeweils einen Phenylrest und die X jeweils einen Sulfonatrest oder einen Carboxylatrest bedeuten. Beispiele für diese Verbindungsklasse der oben wiedergegebenen allgemeinen Formel sind Triphenylphosphin-tri-Natrium-trisulfonat (TPPTS), Triphenylphosphin-tri-(tetraalkylammonium)-trisulfonat, Triphenylphosphin-tri-Natrium-tricarboxylat. Die sulfonierten oder carboxylierten Arylphosphine können als reine Verbindungen eingesetzt werden. Es können aber auch Gemische aus Phosphinen verwendet werden, die unterschiedlich viele Sulfonat- oder Carboxylatgruppen tragen. Besonders bevorzugt kann als wasserlöslicher Organophosphor-Komplexligand Triphenylphosphin-tri-Natrium-trisulfonat nach der Formel (II) eingesetzt werden:

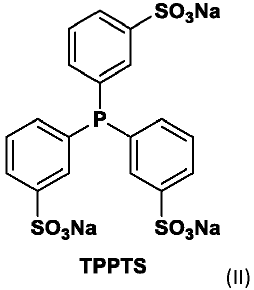

Als wasserlösliche Diphosphine eignen sich ebenfalls sulfonierte Diphosphine der allgemeinen Formeln (III) und (IV)

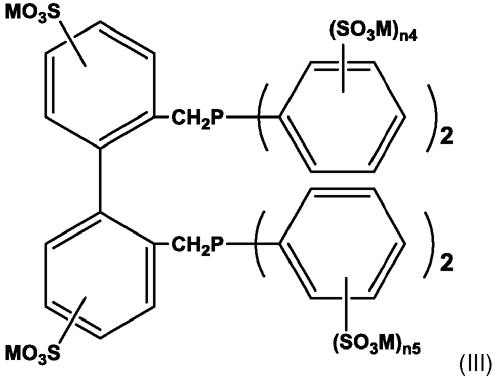

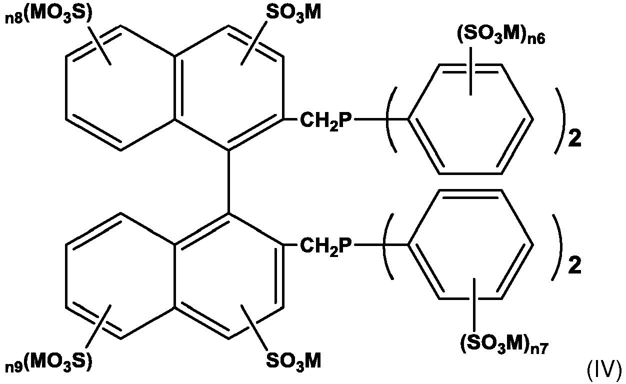

In (III) steht ein jedes n₄ und n₅ unabhängig voneinander für 0 oder 1, wobei die Verbindung der Formel (III) bis sechs -SO₃M-Gruppen enthalten kann.

In (IV) steht ein jedes n₆, n₇, n₈ und n₉ unabhängig voneinander für 0 oder 1, wobei die Verbindung der Formel (IV) vier bis acht -SO₃M-Gruppen enthält.

In den Formeln (III) und (IV) steht M für Ammonium, ein einwertiges Metall oder das Äquivalent eines mehrwertigen Metalls, insbesondere für Natrium, Kalium, Calcium oder Barium.

Im Verfahrensschritt b) wird die Konzentration der Olefine in der Reaktionszone durch Reduzierung der Olefin-Zufuhr zur Reaktionszone um größer oder gleich 50% und kleiner oder gleich 100% bezogen auf die Olefin-Konzentration in der Reaktionszone im Verfahrensschritt a) verringert und mindestens ein Teil der Katalysatorlösung aus dem Reaktionssystem entnommen, wobei die Teilschritte der Katalysatorlösung-Entnahme und der Verringerung der Olefin-Konzentration in dieser oder umgekehrter Reihenfolge, gleichzeitig oder nacheinander erfolgen können. Die Konzentration der Olefine kann beispielsweise durch einen Stopp der Einspeisung und Abreagieren der sich noch im Reaktor befindlichen Verbindungen erreicht werden. Zweckmäßigerweise wird die molare Konzentration der Olefine in diesen Schritt auf 10%, bevorzugt 5%, bevorzugt auf weniger als 1%, bezogen auf die im Verfahrensschritt a) vorherrschende Olefinkonzentration gebracht. Eine Teilmenge an Katalysatorlösung umfasst einen signifikanten Anteil an Katalysatorlösung, welcher größer ist als derjenige Teil, welcher beispielsweise im Rahmen von kontinuierlichen Umlaufverfahren aus der Reaktionszone abgeführt wird. Die Teilmenge kann ein Volumen von mehr als 5%, bevorzugt mehr als 10%, bevorzugt mehr als 15% und des Weiteren bevorzugt mehr als 20% der gesamten wässrigen Katalysatorlösung umfassen. Dieser Verfahrensschritt kann beispielsweise durch das Abführen der Katalysatorlösung eingeleitet werden. Anschließend kann dann die Olefinkonzentration reduziert werden. Bevorzugt kann das Ablassen der Katalysatorlösung aber erst nach der Reduzierung der Olefinkonzentration erfolgen.

Das Reaktionssystem wird dabei aus der Reaktionszone im Reaktor und gegebenenfalls vorliegender, weiterer Einrichtungen, wie dem Katalysator-Kreislaufstrom gebildet. Der Katalysator muss also nicht zwangsläufig direkt vom Reaktionsort abgezogen werden. Es ist möglich, dass der Katalysator im Kreis geführt wird und die Entnahme des Katalysators an einer anderen Stelle des Kreislaufes durchgeführt wird. Diese Maßnahme führt zwangsläufig auch zu einer Reduzierung der Katalysatormenge in der Reaktionszone.

Im Verfahrensschritt c) erfolgt das Zuführen eines Lösemittels, einer Übergangsmetallquelle und wasserlöslicher Organophosphorliganden zum Reaktionssystem, wobei die Zuführung der Komponenten gleichzeitig oder in beliebiger Reihenfolge nacheinander erfolgen kann. Das abgeführte Katalysatorvolumen kann also teilweise, ganz oder in Überschuss durch ein Lösemittel, beispielsweise Wasser, ersetzt werden. Zu der nun verdünnten Katalysatorlösung in der Reaktionszone können dann die Übergangsmetallquelle und daran anschließend der wasserlösliche Organophosphorligand zugeführt werden. Die letzteren Zugaben können beispielsweise in Form konzentrierter wässriger Lösungen mit einem Teil des Lösemittels erfolgen. Es ist aber auch möglich, sämtliche Komponenten, Lösemittel, Übergangsmetallquelle und Komplexligand als eine Lösung dem Reaktionssystem zuzuführen. Das Zusammenführen der Bestandteile kann dabei in der Reaktionszone oder aber im Reaktionssystem erfolgen. Des Weiteren ist es auch möglich den Komplexliganden, zumindest teilweise, schon als Teil der Übergangsmetallquelle mit zuzuführen. Dies bedeutet, dass beispielsweise schon ein präformierter Katalysatorkomplex mit wasserlöslichen Organophosphor-Komplexliganden als Übergangsmetallquelle eingesetzt werden kann. Mögliche nicht präformierte, wasserlösliche Übergangsmetallquellen sind beispielsweise Rhodiumquellen wie beispielsweise [Rh(octanoat)₂]₂, [Rh(acac)(CO)₂], [Rh(2-Ethylhexanoat)₃], [Rh(2-Ethylhexanoat)₂]₂, [Rh(acac)(cod)], [Rh(cod)Cl]₂, [Rh(acac)₃], [Rh(cod)₂]BF₄, [Rh(OAc)₃] oder Mischungen dieser. Nach der Zuführung der Komponenten kann die neue Katalysatorlösung unter Synthesegas für eine gewisse Zeit präformiert werden, wobei die Übergangsmetallquelle unter den angewandten chemischen und physikalischen Bedingungen sowie in Anwesenheit der entsprechenden wasserlöslichen Organophosphorliganden in den aktiven Katalysator überführt wird.

Im Verfahrensschritt d) erfolgt dann die Erhöhung der Konzentration der Olefine in der Reaktionszone durch Erhöhung der Olefin-Zufuhr zur Reaktionszone und Hydroformylierung durch zur Reaktion bringen der Olefine mit Kohlenmonoxid und Wasserstoff. Die Erhöhung der Olefinkonzentration in der Reaktionszone kann dabei durch Starten der Einspeisung des gasförmigen Olefins erfolgen. Zweckmäßigerweise kann die Olefinkonzentration auf denjenigen Wert erhöht werden, wie er im Verfahrensschritt a) vorlag. Es ist aber auch möglich durch die gesteigerte Katalysatoraktivität eine höhere Olefinkonzentration einzuspeisen. Durch die erneute Zufuhr des Olefins zur Reaktionszone werden dann mittels des Synthesegases wieder metallkatalysiert Aldehyde gebildet.

In einer bevorzugten Ausgestaltung des Verfahrens kann im Verfahrensschritt b) der Teilschritt der Verringerung der Olefin-Konzentration vor der Teilentnahme der Katalysatorlösung erfolgen. Es hat sich für die Unterbrechungszeiten der Hydroformylierung als besonders vorteilhaft herausgestellt, dass die Reduzierung der Olefinkonzentration vor der Teilentnahme der Katalysatorlösung erfolgt. Da in dieser Variante eine größere Katalysatorkonzentration vorliegt, kann die Olefinkonzentration im Vergleich zu einer Ausgestaltung, in dem ein Teil der Katalysatorlösung schon entfernt wäre, schneller reduziert werden. Vorteilhafterweise wird in dieser Ausgestaltung weniger Olefin mit der KatalysatorTeilmenge ausgeschleust.

Im Verfahrensschritt b) wird die molare Olefin-Konzentration in der Reaktionszone um größer oder gleich 50% und kleiner oder gleich 100% bezogen auf die Olefin-Konzentration in der Reaktionszone im Verfahrensschritt a) verringert. Zum Erhalt eines möglichst aktiven Katalysators nach der Teilsubstitution der Katalysatorlösung hat es sich als besonders günstig herausgestellt, dass der Olefingehalt in der Reaktionszone signifikant reduziert wird. Bevorzugt kann die Substitution ganz in Abwesenheit von Olefinen in der Reaktionszone erfolgen. Dies kann zu besonders aktiven und selektiven Katalysatoren in der Reaktionszone nach der Substitution führen, welches die Wirtschaftlichkeit des gesamten Verfahrens verbessert. Zur Bestimmung der noch vorliegenden Olefinkonzentration in der Reaktionszone können GC-Methoden herangezogen werden.

Innerhalb eines bevorzugten Aspektes des Verfahrens kann im Verfahrensschritt b) ein Volumen von größer oder gleich 10% und kleiner oder gleich 50% an wässriger Katalysatorlösung dem Reaktionssystem entnommen werden. Zum Erhalt eines möglichst wirtschaftlichen Verfahrens haben sich oben angegebene Teilentnahmemengen als besonders geeignet herausgestellt. Kleinere Entnahmemengen können zu einer nur ungenügenden Wiederherstellung der Katalysatoraktivität und höhere Entnahmemengen zu einer zu großen Störung des Gleichgewichts in der Reaktionszone führen. Bevorzugt kann das Teilentnahmevolumen des Weiteren ≥ 15% und ≤ 45%, weiterhin bevorzugt ≥ 20% und ≤ 40% betragen.

In einer weiteren Ausgestaltung des Verfahrens kann das im Verfahrensschritt c) zugeführte Volumen flüssiger Komponenten größer oder gleich 20% und kleiner oder gleich 200% bezogen auf die im Verfahrensschritt b) entnommene Katalysatorlösung betragen. Zum Erhalt einer sehr aktiven, reaktivierten Katalysatorlösung mit einem relativ geringen Anteil an störenden Katalysatorgiften, hat es sich als besonders geeignet herausgestellt, dass das Volumen der aus der Reaktionszone entfernten Teilentnahmemenge durch das Volumen der neu zugefügten Komponenten annähernd ausgeglichen wird. Eine weitere Verdünnung durch Zugabe eines größeren Volumens, wie oben angegeben, ist üblicherweise innerhalb der angegebenen Grenzen unschädlich und kann zu einer weiteren Reduzierung des Salzgehaltes oder der organischen Komponenten in der wässrigen Katalysatorlösung beitragen. Diese Zugabemengen können zudem weitere Stellkomponenten sein, mit der man auf die erhöhte Effizienz der Katalysatorlösung reagieren kann. Geringere Volumina können nachteilig sein, da dies zu einer nur ungenügenden Verdünnung der Gesamtkatalysatorphase in der Reaktionszone führen kann.

Im Rahmen einer weiteren Ausführungsform des Verfahrens können im Verfahrensschritt c) als Übergangsmetallquelle Rhodium-Verbindungen ausgewählt aus der Gruppe der Rhodium (III)-Salze wie Rh-2-ethylhexanoat, -acetat, -oxalat, -propionat, -malonat, Rh(NO₃)₃, Rh(SO₄)₃, RhCl₃ oder der Rhodiumkomplexverbindungen wie Cyclopentadienylrhodiumverbindungen, [RhCl(Cyclooctadien-1,5)]₂, Rhodiumacetylacetonat, der Rhodiumcarbonylverbindungen wie Rh₃(CO)₁₂, Rh₆(CO)₁₆ oder in Form der verschiedenen Rhodiumoxide oder Mischungen mindestens zweier Verbindungen daraus zugegeben werden. Diese Gruppe an Rh-Verbindungen hat sich als besonders geeignete Rhodiumquelle herausgestellt. Diese Verbindungen lassen sich unter den physikalischen und chemischen Bedingungen des Reaktionssystems oder der Reaktionszone schnell in die gewünschte aktive Katalysatorspezies überführen und liefern nach kurzen Präformierungszeiten hohe Produktivitäten und Selektivitäten. Dies kann zu einer Erhöhung der Wirtschaftlichkeit und der Standzeit des Verfahrens beitragen. Insbesondere kann man sich durch den Einsatz dieser Rhodiumquellen weitere Aufbauten zur Präformierung des Katalysators sparen, da die Formierung des Katalysators, also die Umwandlung in den aktiven Katalysator mit Wasserstoff, Kohlenmonoxid und wasserlöslichen Organophosphor-Komplexliganden, in der Reaktionszone selbst stattfindet.

Im Rahmen eines weiteren, bevorzugten Aspektes des Verfahrens können im Verfahrensschritt c) als Übergangsmetallquelle präformierte Rhodium-Komplexe mit Organophosphorliganden zum Reaktionssystem zugegeben werden. Zur Verkürzung der Präformierungszeit des Katalysators in der Reaktionszone hat es sich als günstig herausgestellt, dass als Rhodiumquelle schon präformierte Katalysatoren der Reaktionszone zugeführt werden. In dieser findet dann eine schnellere Gleichgewichtseinstellung und Adaption auf die Reaktionsbedingungen statt. Auf diese Art können sehr kurze Unterbrechungen der Synthese zur Substitution der Katalysatorlösung gewährleistet werden.

Innerhalb einer weiteren Ausgestaltung des Verfahrens können im Verfahrensschritt c) Organophosphorliganden in einem molaren Verhältnis von größer oder gleich 20 und kleiner oder gleich 400 bezogen auf die in Verfahrensschritt b) entnommene Übergangsmetallmenge zugegeben werden. Zur schnellen Einstellung einer möglichst aktiven Katalysatorgesamtheit in der Reaktionszone hat sich der Zusatz oben angegebener Liganden als besonders geeignet herausgestellt. Diese Menge kann zu einer besonders schnellen Präformierung der durch die Zugabe von Organophosphorliganden neu gebildeten Rhodium-Phosphin-Komplexe und, darüber hinaus, zu einer Reaktivierung der in der Reaktionszone verbliebenen Katalysatoren beitragen. Letztere wurden ggf. durch Katalysatorgifte in ihrer Syntheseleistung und/oder Selektivität beeinträchtigt. Höhere Verhältnisse können zu einer zu hohen organischen/Salz-Belastung der wässrigen Phase und niedrigere Verhältnisse zu einer nur ungenügenden Reaktivierung der Katalysator-Metallzentren führen.

Innerhalb einer bevorzugten Ausführungsform des Verfahrens kann im Verfahrensschritt c) eine Lösung einer Rhodium-Verbindung umfassend Organophosphorliganden dem Reaktionssystem zugeführt werden. Zur Vereinfachung der Zugabeschritte und zur schnellen Gleichgewichtseinstellung, hat sich eine gleichzeitige Zugabe einer nicht präformierten Lösung einer Rhodium-Verbindung, welche zudem wasserlösliche Organophosphorliganden aufweist, als besonders geeignet herausgestellt. Durch diese Verfahrensführung entfallen weitere technische Aufbauten, um eine Präformierung des Katalysators zu erreichen. Des Weiteren kann die Präformierung in der Reaktionszone im Vergleich zu einer getrennten Zugabe der Komponenten schneller erfolgen. Bevorzugt kann die Zugabe auch direkt zur Reaktionszone erfolgen.

In einem bevorzugten Aspekt des Verfahrens können im Verfahrensschritt c) vor der Zuführung der Übergangsmetallquelle Organophosphorliganden dem Reaktionssystem zugeführt werden. Sollte die Katalysatorlösung schon einen hohen Grad an Alterungserscheinungen, wie beispielsweise ein ungünstig verändertes/verschlechtertes Isomerenverhältnis oder eine nur sehr ungenügende Aktivität zeigen, kann es sich als günstig herausstellen, dass vor der Zugabe der Übergangsmetallquelle erst einmal die in der Reaktionszone verbleibende Restmenge an Katalysatorlösung mit einem wasserlöslichen Organophosphorliganden in Kontakt gebracht wird, bevor die frische Übergangsmetallquelle hinzugefügt wird. Diese Verfahrensführung kann zu einer selektiveren Aktivierung des verbliebenen Katalysators beitragen und man erhält, im Vergleich mit einer gleichzeitigen Zugabe von Übergangsmetallquelle und Organophosphorliganden, in Summe einen aktiveren und selektiveren Katalysator in der Reaktionszone. Die Zugabe der Organophosphorliganden kann dabei direkt in die Reaktionszone oder beispielsweise über die Zugabe in den Katalysatorkreislauf in das Reaktionssystem erfolgen.

Nach einer weiteren, bevorzugten Ausführungsform des Verfahrens kann im Verfahrensschritt c) zuerst eine Teilmenge des Lösemittels, dann die in einem Lösemittel gelöste Übergangsmetallquelle und anschließend die Organophosphorliganden dem Reaktionssystem zugeführt werden. Für eine gesteuerte Reaktionsführung hat es sich als besonders geeignet herausgestellt, dass die Zugabe in diesem Verfahrensschritt in oben angegebener Reihenfolge abläuft. Durch die sequentielle Zugabe kann immer erst eine verdünnte wässrige Katalysatorlösung in der Reaktionszone erhalten werden, zu welcher dann die weiteren Komponenten hinzugegeben werden. Durch geeignete technische Maßnahmen oder durch die Synthesegas-Konvektion der Lösung in der Reaktionszone kann die Gleichgewichtseinstellung zudem beschleunigt werden.

Nach einer bevorzugten Charakteristik des Verfahrens kann durch die Verfahrensschritte b) und c) die Gesamtkonzentration vorliegender organischer Liganden und Salze in der wässrigen Lösung der Reaktionszone um größer oder gleich 10% und kleiner oder gleich 50% im Vergleich zu der Konzentration der organischen Liganden und der Salze in der wässrigen Lösung der Reaktionszone am Ende des Verfahrensschrittes a) reduziert werden. Überraschenderweise hat sich gezeigt, dass das erfindungsgemäße Verfahren in der Lage ist, den Salzgehalt und/oder die organische Fracht in der wässrigen Katalysatorlösung der Reaktionszone deutlich zu reduzieren. Insbesondere kann die Konzentration der LigandenAbbauprodukte reduziert werden, wodurch sich die Langlebigkeit des Katalysators in der Reaktionszone insgesamt erhöht. Die Menge an phosphororganischen Liganden in der wässrigen Lösung kann beispielsweise über HPLC-Messungen bestimmt werden. Insbesondere kann der Anteil an TPPTS-Abbauprodukten reduziert werden, welches zu einer Verbesserung des Isomerenverhältnisses führt. Zu den organischen Liganden zählen dabei insbesondere Komplexbildner mit einem Kohlenstoffgerüst und Heteroatomen, wobei die Komplexbildung über die Heteroatome verläuft. Die organischen Liganden umfassen also die gewünschten, funktionalen Organophosphorliganden sowie deren Abbauprodukte. Zusätzlich zählen zu der Gruppe der organischen Liganden auch die Liganden der wasserlöslichen, zugesetzten Übergangsmetallquellen, beispielsweise in Form von Acetat oder ähnlichen Verbindungen mit Kohlenstoff- und Heteroatomanteil.

Innerhalb einer weiteren Ausführungsform des Verfahrens kann im Verfahrensschritt c) der pH-Wert in der wässrigen Lösung der Reaktionszone nach Zugabe der Komponenten auf einen Bereich von größer oder gleich pH 4 und kleiner oder gleich pH 10 eingestellt werden. Zum Erhalt einer möglichst hohen Katalysatoraktivität hat es sich zudem als hilfreich herausgestellt, nach der Zugabesequenz den pH-Wert der Katalysatorlösung in der Reaktionszone einzustellen. Dies kann durch bekannte Stellmittel wie anorganische Säuren oder Basen erfolgen. Es ist auch möglich, die neu zugegebene Katalysatormenge schon mit diesen zu versehen und so eine Beschleunigung der Prozess-/Reaktivierungszeiten zu erreichen. In einer weiter bevorzugten Ausführungsform kann der pH-Wert zwischen größer oder gleich pH 5 und kleiner oder gleich pH 8, des Weiteren bevorzugt zwischen größer oder gleich pH 5,5 und kleiner oder gleich pH 7 eingestellt werden.

In einer weiteren Ausgestaltung des Verfahrens kann das molare Verhältnis von zugegebenen Organophosphorliganden zu zugegebenen Übergangsmetall im Verfahrensschritt c) größer oder gleich 20 und kleiner oder gleich 500 betragen. Zur Stabilisierung der Übergangsmetallquelle und zur Beschleunigung der Gleichgewichtseinstellung hat es sich als günstig herausgestellt, dass der wasserlösliche Organophosphorligand in diesem Schritt in einem hohen Überschuss eingesetzt wird. Bevorzugte Verhältnisse liegen des Weiteren zwischen größer oder gleich 120 und kleiner oder gleich 450 und bevorzugt bei größer oder gleich 150 und kleiner oder gleich 400.

Nach einer weiter bevorzugten Ausführungsform des Verfahrens kann durch die Verfahrensschritte b) und c) die Konzentration der anorganischen Kationen in der Lösung der Reaktionszone um größer oder gleich 5% reduziert werden. Es hat sich zudem als sehr vorteilhaft herausgestellt, dass neben der Konzentration organischer Liganden in der Reaktionszone auch die Menge an anorganischen Kationen reduziert wird. Zu den anorganischen Kationen gehören beispielsweise Alkali- und Erdalkali-lonen. Ohne durch die Theorie gebunden zu sein können diese Ionen anscheinend entweder Teil der Katalysatorkomplexe sein oder aber durch Änderung des Verteilungsgleichgewichtes des Olefins zwischen wässriger und organischer Phase zu einer Verbesserung der Löslichkeit der Olefine in die wässrige Phase beitragen. Durch eine Verringerung der Konzentration der Kationen wird also die Polarität der wässrigen Phase herabgesetzt und es kann ein vorteilhafteres Phasengleichgewicht für das Olefin erreicht werden. Letzteres kann insbesondere zu einer Steigerung der Reaktionsgeschwindigkeit beitragen. In besonders bevorzugten Ausführungsformen kann die Konzentration der anorganischen Kationen in der Lösung der Reaktionszone um größer oder gleich 10%, besonders bevorzugt um größer oder gleich 15% reduziert werden. Diese Bereiche der Reduzierung der Kationen können zu einer besonders effizienten Wiederherstellung der Katalysatoraktivität beitragen. Die Messung der Konzentrationen kann beispielsweise über ICP-Methoden erfolgen.

Weitere Einzelheiten, Merkmale und Vorteile des Gegenstandes der Erfindung ergeben sich aus den Unteransprüchen sowie aus der nachfolgenden Beschreibung der Figuren und der zugehörigen Beispiele. Es zeigt die:
- Fig. 1: schematisch die Durchführung eines kontinuierlichen Ruhrchemie-/Rhone-Poulenc-Hydroformylierungsverfahren;
- Fig. 2: schematisch den Verfahrensablauf bei der Durchführung des erfindungsgemäßen Hydroformylierungsverfahren.

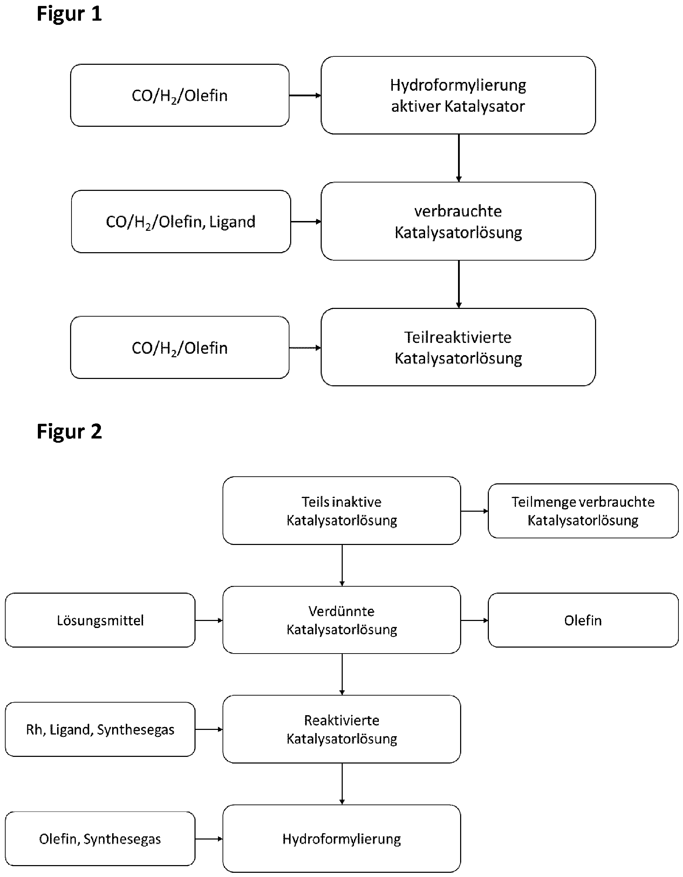

Die Figur 1 zeigt schematisch die Durchführung eines kontinuierlichen Ruhrchemie-/Rhone-Poulenc-Hydroformylierungsverfahren. Zu einer wässrigen, aktiven Katalysatorlösung in einer Reaktionszone werden kontinuierlich das Olefin-Edukt und Synthesegas zugeführt.

Durch die angewandten Reaktionsbedingungen kommt es zu einem chemischen Abbau des Katalysators, wodurch sich die Syntheseleistung und die Selektivität der Umsetzung verschlechtern. Als Reaktion auf die Verschlechterung wird in zeitlichen Abständen zusätzlicher Ligand, ein wasserlöslicher Organophosphor-Komplexligand, wie beispielsweise TPPTS, zur Reaktionszone gegeben. Aufgrund der Gleichgewichtsreaktion mit dem neu hinzugefügten Liganden und unter partieller Verdrängung der Katalysatorgifte aus den Metallkomplexen verbessert sich sowohl die Syntheseleistung als auch das Isomerenverhältnis. Die Hydroformylierung wird dann wieder wie gewohnt unter Zuführung von Synthesegas und Olefin fortgesetzt. Nach mehrmaligen Zugaben des wasserlöslichen Organophosphor-Komplexliganden ist die Salz- und die organische Fracht in der Katalysatorlösung allerdings so hoch, dass die Reaktion beendet und die gesamte Katalysatorlösung aufgearbeitet werden muss.

Die Figur 2 stellt schematisch den Verfahrensablauf des erfindungsgemäßen Verfahrens dar. Nach einer gewissen Laufzeit der Hydroformylierung ergibt sich wie in dem Stand der Technik Verfahren eine partielle Deaktivierung der Metallkatalysatoren, welches sich durch eine verschlechterte Ausbeute und/oder ein verschlechtertes Isomerenverhältnis bemerkbar macht. In diesem Fall wird eine Teilmenge der Katalysatorlösung aus dem Reaktionsort oder dem Reaktionssystem ausgeschleust. Die ausgeschleuste Teilmenge wird durch Lösungsmittel ersetzt und man erhält eine verdünnte Katalysatorlösung. Zudem wird gleichzeitig, vorab oder nach dem Ausschleusen, die Olefinmenge am Reaktionsort um größer oder gleich 50% und kleiner oder gleich 100% bezogen auf die Olefin-Konzentration in der Reaktionszone im Verfahrensschritt a) reduziert. Letzteres kann beispielsweise durch ein Stoppen der Olefin-Zufuhr und ein Ausreagieren des noch in der Reaktionszone vorhandenen Olefins erreicht werden. Zu der Olefin-verarmten Reaktionszone werden nun gleichzeitig oder nacheinander eine Rhodiumquelle, und weitere, wasserlösliche Organophosphor-Komplexliganden zugeführt. Diese supplementierte Katalysatorlösung wird unter Abwesenheit von Olefinen und Anwesenheit von Synthesegas zu den aktiven Katalysatoren präformiert. Nach der Präformierung wird die Olefinzufuhr wieder gestartet und die Hydroformylierung erfolgt mit einer deutlich besseren Syntheseleistung und einem verbesserten Isomerenverhältnis. Auf diese Art und Weise kann die kontinuierliche Hydroformylierungsreaktion, verglichen mit den Stand-der-Technik-Verfahren, über einen deutlich längeren Zeitraum gleichbleibend fortgeführt werden.

### Beispiele

Die Vorteile des erfindungsgemäßen Verfahrens werden anhand der folgenden Beispiele aufgezeigt.

### Beispiel 1 (nicht erfindungsgemäß, Zugabe erfolgt in Gegenwart von Olefin)

Eine gebrauchte wässrige Katalysatorlösung (Rh mit TPPTS als Organophosphorliganden und einen Ligandenverhältnis P(III):Rh von ca. 90:1) wurde in einer halbkontinuierlich arbeitenden Apparatur eingesetzt und bei 50 bar Synthesegasdruck, 137°C, pH 6,0 und einem Propylen-Einsatz von 60 g/h über 14 h betrieben. Man erhält als Maß für die Produktivität des Katalysators einen p-Wert von 0,272 (kg_{Aldehyd}/L_{Katlsg.}x h) als Ausgangsniveau. Anschließend wurde in zwei Schritten 20% bzw. 35% der Katalysatorlösung entnommen und jeweils mit der entsprechenden Menge deionisiertem Wasser ersetzt. Obwohl die Rhodiummenge am Reaktionsort durch diese beiden Teilausschleusungen annähernd halbiert wurde, sank der p-Wert lediglich um ca. 20%. Durch die Verdünnung der Katalysatorlösung sank allerdings die Regioselektivität in Bezug auf das *n*-Aldehyd von 91% auf 89%.

Die ursprüngliche Rhodiummenge im Reaktor wurde anschließend durch Zugabe von Rhodium(III)-Acetat während der laufenden Hydroformylierung, also unter Zuführung von Synthesegas und Einsatzolefin, wiederhergestellt. Durch die Zugabe von Rhodium(III)-Acetat erfolgte eine Absenkung des P(III):Rh-Verhältnisses (219 vs. 112 ppm bzw. 74:1 vs. 43:1), wobei die Absenkung des Ligandenverhältnisses weder Auswirkungen auf die Produktivität noch Selektivität zeigte. Eigentlich sollte es zu einer deutlichen Aktivitätszunahme durch die Zugabe weiteren Rhodiums kommen. Des Weiteren sollte die Regioselektivität durch das niedrigere P(III):Rh-Verhältnis weiter absinken. Es ergaben sich für den Versuch folgende Werte:

**Tabelle 1: Beispiel 1 - Entnahme und Zugabe unter Standardbedingungen, insbesondere in Gegenwart von Olefin. 50 bar, 60 g/h Propyleneinsatz, pH 6,0, n-C₄-al wird berechnet als nl(n+i)**

| Schritt | | Vol. [%] | Rh [ppm] | P(III):Rh | T [°C] | p [kg/L x h] | *n*-C₄-al [%] |
|---|---|---|---|---|---|---|---|
| 1 | Null-Niveau | - | 223 | 89 | 137 | 0,272 | 90,8 |
| 2 | 1. Entnahme | 20 | 193 | 80 | 137 | 0,240 | 90,9 |
| 3 | 2. Entnahme | 35 | 112 | 75 | 137 | 0,223 | 88,5 |
| 4 | Rh-Zugabe | - | 219 | 43 | 137 | 0,224 | 88,5 |

Durch obige Tabelle wird deutlich, dass die Supplementierung des entnommenen Rhodiums, ohne Zurückführen der Olefinkonzentration in der Reaktionszone, nur zu einem ungenügenden Wiederanstieg der Syntheseleistung und zu keiner Verbesserung des n:i Isomerenverhältnisses führt. Diese Werte verbessern sich auch nicht als Funktion der Zeit, sodass dieser Effekt nicht auf kinetische Effekte, wie beispielsweise eine ungenügende Zeitspanne zur Bildung der eigentlichen aktiven Katalysatorspezies, zurückgeführt werden kann. Vermutlich konnte das als Rhodium(III)-Acetat eingebrachte Metall unter den Reaktionsbedingungen überhaupt nicht oder nur zu einem ungenügenden Teil in den katalytisch aktiven Rhodium(I)-Komplex überführt werden. Eventuell ergibt sich aber auch eine verstärkte Deaktivierung des neu zugesetzten Rhodiums durch eine ungewünschte und konkurrierende Komplexbildung mit koordinierenden Ligandenabbauprodukten. Unter Umständen könnte das zugegebene Rhodium(III) mit den in der Reaktionszone vorliegenden Olefinen stabile Rhodium(III)-Alkenyl-Komplexe ausbilden, welche hydroformylierungsinaktiv sind und unter den Bedingungen in der Reaktionszone nicht in katalytisch aktive Rhodium(I)-Komplexe überführt werden können.

### Beispiel 2 (erfindungsgemäßer Prozess, Ergänzung in Abwesenheit von Olefinen)

Bei 126°C, 50 bar, einem Propylen-Einsatz von 60 g/h und einem pH von 6,0 wurde über ca. 23 h die Grundleistung der gebrauchten Katalysatorlösung mit p = 0,155 (kg_{Aldehyd}/L_{Katlsg.}x h). und 90,9% n-Aldehydanteil bestimmt. Anschließend wurde 21% der Katalysatorlösung entnommen und der Volumenverlust mit Wasser ausgeglichen. Der p-Wert sank auf 0,138 (kg_{Aldehyd}/L_{Katlsg}.x h) und der *n*-Aldehydanteil auf 90,0%. Nach 36 h wurde die Anlage auf 10 bar entspannt und anschließend mit Synthesegas ein Druck von 50 bar eingestellt, wobei das im Reaktor vorhandene Propylen entfernt wurde. Entsprechend der zuvor entnommenen Katalysatormenge wurde Rhodium(III)-Acetat zugegeben und die Katalysatorlösung 3 h bei 50 bar Synthesegasdruck sowie 121°C gerührt. Anschließend wurde die Propylenzugabe wieder gestartet. Nach dieser Prozedur wurde ein deutlich erhöhter p-Wert von 0,169 (kg_{Aldehyd}/L_{Katlsg.}x h) erhalten. Dieser Wert für die Syntheseleistung liegt über denen des Ausgangsniveaus und ist für einen gebrauchten und aufgefrischten Katalysator bei 126°C hoch. Der p-Wert erlaubt eine Aussage über die erzielte Produktmenge pro Katalysatorvolumen und Zeit. Die Selektivität sank allerdings auf 89,8% *n*-Aldehyd. Nach 48 h wurde dem System der Ligand TPPTS zugegeben, um das P(III):Rh auf ein Verhältnis von > 90:1 zu steigern. Das durch das TPPTS eingebrachte Volumen hatte eine starke Verdünnung des Katalysatorkreislaufs und entsprechend der Rhodiumkonzentration zur Folge, was zu einer Abnahme von p-Wert auf p = 0,130 (kg_{Aldehyd}/L_{Katlsg}.x h) führte. Berücksichtigt man die Verdünnung ergibt sich aber im Vergleich zum Beispiel 1 ein deutlich aktiverer Katalysator. Die Selektivität stieg zudem auf *n*-Anteil von 90,7%. Die Abwesenheit von Propylen während der Zugabe scheint also für eine effektive Rhodium(III)-Addition entscheidend zu sein, da im Gegensatz zum Beispiel 1 sich höhere Produktivität und ein verbessertes Isomerenverhältnis nach der Zugabe einstellen. Zudem kann die Temperatur nach Supplementierung wieder abgesenkt werden, welches im weiteren Verfahrensverlauf wieder die Katalysatorstandzeit erhöhen kann.

**Tabelle 2: Beispiel 2 - Entnahme und Zugabe unter modifizierten Bedingungen; 50 bar, 60 g/h Propyleneinsatz, pH 6,0, n-C₄-al wird berechnet als nl(n+i)**

| Schritt | | Vol. [%] | Rh [ppm] | P(III):Rh | T [°C] | p [kg/L x h] | *n*-C₄-al [%] |
|---|---|---|---|---|---|---|---|
| 1 | Null-Niveau | - | 293 | 36 | 126 | 0,155 | 90,9 |
| 2 | Entnahme | 21 | 224 | 36 | 126 | 0,138 | 90,0 |
| 3 | Rh-Zugabe | - | 284 | 15 | 126 | 0,169 | 89,8 |
| 4 | TPPTS-Zugabe | - | 256 | 120 | 126 | 0,130 | 90,7 |

### Beispiel 3 (erfindungsgemäß)

Die im Beispiel 2 beschriebene Prozedur wurde in einem weiteren Experiment bei 50 bar, 126°C, 140 g/h Propyleneinsatz und pH 6, vier Mal wiederholt. Die Zugabe der neuen Katalysatorlösung erfolgte immer in Abwesenheit von Edukt-Olefin. Das Hydroformylierungs-Grundniveau wurde zu p = 0,166 (kg_{Aldehyd}/L_{Katlsg}.x h) und 92,5% *n*-C4-al bestimmt. Die Aktivität der Katalysatorlösung übertraf nach der Supplementierung des entfernten Katalysatorvolumens das Grundniveau und erhöhte sich im Schnitt auf p = 0,178 (kg_{Aldehyd}/L_{Katlsg.}x h). Die Regioselektivität nahm nach der 3. Sequenz bis auf 90,6% n-Anteil bei P(III):Rh von 60:1 ab und wurde in der 4. Sequenz durch weitere Ligandenzugabe auf ein Verhältnis von 80:1 gesteigert, welches eine Selektivität von 91,1% ergab.

**Tabelle 3.1: Entnahme und Zugabe unter vollkontinuierlichen Bedingungen, Präformierung im Reaktor 3 h, 50 bar, 140 g/h Propyleneinsatz, pH 6,0, n-C₄-al wird berechnet als nl(n+i)**

| Schritt | | Vol. [%] | Rh [ppm] | P(III):Rh | T [°C] | p [kg/L x h] | *n*-C₄-al [%] |
|---|---|---|---|---|---|---|---|
| 1 | Null-Niveau | - | 245 | 88 | 126 | 0,166 | 92,5 |
| 2 | 1. Sequenz | 20 | 236 | 82 | 126 | 0,179 | 91,6 |
| 3 | 2. Sequenz | 20 | 218 | 72 | 126 | 0,180 | 91,1 |
| 4 | 3. Sequenz | 20 | 210 | 60 | 126 | 0,176 | 90,6 |
| 5 | 4. Sequenz | 20 | 235 | 80 | 126 | 0,178 | 91,1 |

Da die Teilsubstitution auf eine Aktivitätssteigerung der eingesetzten Katalysatorlösung durch Austrag desaktivierender Komponenten abzielt, wurden ebenfalls die Konzentrationen entsprechender Zersetzungsprodukte verfolgt (*Tabelle 3.2).* Vor der ersten Katalysatorentnahme betrugen die Konzentrationen von TPPOTS 4,4% und eines der Ligandenabbauprodukte (K5) 7,9%. Zu den Zersetzungsprodukten gehören also alle diejenigen organischen Verbindungen in der Reaktionslösung, welche zumindest ein Teil des Grundgerüstes des eingesetzten organischen Liganden aufweisen und über die in der Reaktionszone vorherrschenden chemischen Bedingungen, zumindest in Teilen, chemisch modifiziert wurden. Zu den chemischen Modifikationen der eingesetzten Liganden können beispielsweise ein oder mehrmalige Oxidations-/Reduktionsprozesse sowie Abspaltungen einer oder mehrerer funktionaler- oder CH-Gruppen gehören.

Der Gesamtsalzgehalt der Katalysatorlösung, gemessen nach HPLC Analyse, lag bei 29,1%. Nach der ersten Substitutionssequenz inklusive der Rhodium- und TPPTS-Ergänzung lagen die Konzentrationen von TPPOTS bei 3,5% und von K5 bei 6,1%. Der Gesamtsalzgehalt lag bei 23,2% und insofern signifikant niedriger als beim Ausgangsniveau. Der TPPTS-Gehalt lag durch die Ergänzung bei 9,8% gegenüber zuvor 11,8%.

**Tabelle 3.2: Beispiel 3 - Änderung der Salzgehalte nach den Katalysatorteilsubstitutionen.**

| Eintrag | Salz | Start | 1. | Δ [%] | 2. | Δ [%] | 3. | Δ [%] | 4. | Δ [%] |
|---|---|---|---|---|---|---|---|---|---|---|
| 1 | OTS | 4.4 | 3.5 | -20.5 | 3.1 | -11.4 | 2.3 | -25.2 | 1.8 | -21.8 |
| 2 | K5 | 7.9 | 6.1 | -22.8 | 5.7 | -6.6 | 4.7 | -17.6 | 4.0 | -14.9 |
| 3 | TS | 11.8 | 9.8 | -16.9 | 9.3 | -5.1 | 7.2 | -22.6 | 8.1 | +12.5 |
| 4 | Ges. | 29.1 | 23.2 | -20.3 | 21.7 | -6.5 | 17.1 | -21.2 | 16.4 | -4.1 |

Insgesamt konnten in der ersten Substitution die Konzentrationen der Salze um > 20% verringert werden. Der TPPTS-Gehalt hingegen wurde durch die Zusätze relativ stabil gehalten. Für die Daten ist zu beachten, dass die P(III)-Zersetzung während der ersten Substitutionssequenzen nicht ausgeglichen wurde. Erst in der letzten Sequenz wurde ein deutlicher Überschuss TPPTS zugesetzt, so dass der TPPTS-Gehalt auf 12,5% bzw. ein P(III):Rh Verhältnis von > 80:1 gebracht wurde.

### Beispiel 4 (Zusammenführung der einzelnen Metallquellen und Ligandenzugabe)

In diesem Versuch wurde bei 139°C, 50 bar, einem Propyleneinsatz von 145 g/h und einem pH-Wert von 6,0 über 427 h die Aktivitäts- und Selektivitätsabnahme einer Katalysatorlösung verfolgt, welche ein sehr niedriges P(III):Rh-Verhältnis von < 50:1 aufwies. Der p-Wert sank innerhalb der Reaktionszeit von 0,237 (kg_{Aldehyd}/L_{Katlsg.}x h) stetig bis auf 0,123 (kg_{Aldehyd}/L_{Katlsg.}x h). Anschließend wurde die Substitution von 20% des Katalysatorvolumens eingeleitet. Der Propyleneinsatz wurde gestoppt, das im Reaktor befindliche Propylen umgesetzt und dem Katalysatorkreislauf 20% des Katalysatorvolumens entnommen. Das fehlende Katalysatorvolumen wurde direkt mit einer entsprechenden Menge an Rhodium(III)-Acetat gelöst in einer wässrigen TPPTS-Lösung (ca. 30 Gew-%; resultierend in P(III):Rh = 102:1) ersetzt. Bei Reaktionstemperatur (138°C) und unter einer Synthesegasatmosphäre (50 bar) wurde das annähernd propylenfreie Reaktionsgemisch 3 h gerührt bevor dann die Propylenzufuhr langsam gestartet und bis auf 145 g/h gesteigert wurde. In den ersten 20 h nach dem Ersatz steigerte sich der p-Wert von 0,123 (kg_{Aldehyd}/L_{Katlsg}.x h) auf 0,184 (kg_{Aldehyd}/L_{Katlsg.}x h) und in den darauffolgenden 24 h weiter auf 0,209 (kg_{Aldehyd}/L_{Katlsg.}x h). Da die Substitution hier in einem Schritt, also durch gleichzeitige Zugabe der Rhodiumquelle in einer Lösung mit oder unter gleichzeitiger Zugabe von Liganden durchgeführt wurde, konnte darüber hinaus auf eine vorherige Stabilisierung der Katalysatorlösung durch TPPTS verzichtet werden. Die Ergebnisse dieser Sequenz ergeben sich wie folgt:

**Tabelle 4: Substitution an einer inaktiven Katalysatorlösung (vollkontinuierlich). 50 bar, 145 g/h Propyleneinsatz, pH 6,0, n-C₄-al wird berechnet als nl(n+i)**

| Schritt | | Vol. [%] | Rh [ppm] | P(III):Rh | T [°C] | p [kg/L x h] | *n*-C₄-al [%] |
|---|---|---|---|---|---|---|---|
| 1 | Start | - | 271 | 44 | 139 | 0,234 | 89,5 |
| 2 | Ende | - | 274 | 28 | 139 | 0,123 | 89,8 |
| 5 | Ergänzung | 20 | 268 | 99 | 139 | 0,209 | 89,0 |

### Beispiel 5 (gealterte Katalysatorlösung)

Es wurde in einer bevorzugten Verfahrensvariante versucht die Substitutionssequenz an einer sehr stark gealterten Katalysatorlösung durchzuführen. Bei 50 bar, 138°C, 140 g/h Propyleneinsatz und einem pH-Wert von 6,0 wurde das Grundniveau der Umsetzung bestimmt und die Substitutionssequenz mit der Entnahme und Verdünnung begonnen. Hierbei brach die Hydroformylierung zusammen und das Experiment musste abgebrochen werden. Eine Reaktivierung des Katalysators war nicht möglich.

**Tabelle 5: Beispiel 5 - Substitution an einer inaktiven Katalysatorlösung (vollkontinuierlich). 50 bar, 140 g/h Propyleneinsatz, pH 6,0, n-C₄-al wird berechnet als n/(n+i)**

| Schritt | | Vol. [%] | Rh [ppm] | P(III):Rh | T [°C] | p [kg/L x h] | *n*-C₄-al [%] |
|---|---|---|---|---|---|---|---|
| 1 | Null-Niveau | - | 247 | 42 | 138 | 0,147 | 90,2 |
| 2 | Entnahme | 20 | 185 | 35 | 138 | 0,078 | 90,2 |

Liegt in einer sehr stark gealterten Katalysatorlösung schon ein niedriges Liganden-zu-Rhodiumverhältnis vor, kann die alleinige Substitution des Metalls die Katalysatorlösung nicht reaktivieren. In diesen Fällen sind die Liganden der bestimmende Faktor und nicht das Metall. Dennoch lässt sich über das erfindungsgemäße Verfahren eine Reaktivierung erreichen. Ein mögliches Verfahren für diese Fälle ist im Beispiel 6 wiedergegeben.

### Beispiel 6 (gealterte Katalysatorlösung)

Unter modifizierten Bedingungen wurde der in Beispiel 5 eingesetzten Katalysatorlösung zunächst weiterer Ligand zugesetzt und damit das System stabilisiert (50 bar, 136°C, 40 g/h Propyleneinsatz, pH-Wert von 6,0). Bereits durch die alleinige Zugabe an Ligand war eine deutliche Aktivitäts- sowie Selektivitätszunahme zu beobachten, welches belegt, dass die Ligandenkonzentration den bestimmenden Faktor für die Produktivität des Katalysators darstellt. Nach der Stabilisierung durch Ligandenzugabe wurde die erfindungsgemäße Substitutionssequenz zweimal durchgeführt, wobei die Präformierungszeit jeweils 3 h betrug. Die Rücknahme der Temperatur von 136°C auf 131°C nach Stabilisierung sowie die jeweilige mögliche Steigerung des Olefineinsatzes nach den Substitutionen (40 g/h ----> 45 g/h → 55 g/h) sind Zeichen einer deutlichen Katalysatoraktivitätszunahme. Im Ergebnis zeigt sich, dass sehr inaktive Katalysatorlösungen ebenfalls einer Substitution und Reaktivierung unterzogen werden können. Dazu müssen diese speziellen Katalysatorlösungen allerdings zuvor durch weitere Ligandenzugabe stabilisiert werden. Die Ergebnisse der Substitution sind in der Tabelle 6 dargestellt.

**Tabelle 6: Beispiel 6 - Substitution an einer inaktiven Katalysatorlösung nach vorheriger Stabilisierung (vollkontinuierlich), n-C4-al wird berechnet als nl(n+i)**

| Schritt | | Vol. [%] | Rh [ppm] | P(III):Rh | Propylen-einsatz [g/h] | T [°C] | P [kq/L × h] | *n*-C₄-al [%] |
|---|---|---|---|---|---|---|---|---|
| 1 | Null-Niveau | - | 253 | 46 | 40 | 136 | 0,162 | 87,7 |
| 2 | Stabilisation | - | 236 | 67 | 40 | 136 | 0,197 | 90,9 |
| 3 | Stabilisation | - | 236 | 67 | 40 | 131 | 0,145 | 90,9 |
| 4 | 1. Sequenz | 20 | 248 | 57 | 45 | 131 | 0,163 | 90,2 |
| 5 | 2. Sequenz | 20 | 240 | > 42 | 55 | 131 | 0,158 | 90,9 |

## Patentansprüche

1. Kontinuierliches Zwei-Phasen Hydroformylierungsverfahren zur Darstellung von Aldehyden aus Olefinen mittels Kohlenmonoxid, Wasserstoff und einem Übergangsmetallkatalysator in einer Reaktionszone, wobei das Übergangsmetall in Form eines wasserlöslichen Katalysatorkomplexes vorliegt, **dadurch gekennzeichnet, dass** das Verfahren ein oder mehrmals die folgenden Schritte umfasst:
a) Hydroformylieren durch zur Reaktion bringen der Olefine, Kohlenmonoxid und Wasserstoff an einem wasserlöslichen Übergangsmetallkatalysator aufweisend wasserlösliche Organophosphorliganden in einer Reaktionszone;
b) Verringerung der Konzentration der Olefine in der Reaktionszone durch Reduzierung der Olefin-Zufuhr zur Reaktionszone um größer oder gleich 50% und kleiner oder gleich 100% bezogen auf die Olefin-Konzentration in der Reaktionszone im Verfahrensschritt a) und Entnehmen mindestens eines Teils der Katalysatorlösung aus dem Reaktionssystem, wobei die Teilschritte der Katalysatorlösung-Entnahme und der Verringerung der Olefin-Konzentration in dieser oder umgekehrter Reihenfolge, gleichzeitig oder nacheinander erfolgen können;
c) Zuführen eines Lösemittels, einer Übergangsmetallquelle und wasserlöslicher Organophosphorliganden zum Reaktionssystem, wobei die Zuführung der Komponenten gleichzeitig oder in beliebiger Reihenfolge nacheinander erfolgen kann;
d) Erhöhung der Konzentration der Olefine in der Reaktionszone durch Erhöhung der Olefin-Zufuhr zur Reaktionszone und Hydroformylieren durch zur Reaktion bringen der Olefine mit Kohlenmonoxid und Wasserstoff.

2. Verfahren nach Anspruch 1, wobei im Verfahrensschritt b) der Teilschritt der Verringerung der Olefin-Konzentration vor der Teilentnahme der Katalysatorlösung erfolgt.

3. Verfahren nach einem der vorhergehenden Ansprüche, wobei im Verfahrensschritt b) ein Volumen von größer oder gleich 10% und kleiner oder gleich 50% an wässriger Katalysatorlösung dem Reaktionssystem entnommen wird.

4. Verfahren nach einem der vorhergehenden Ansprüche, wobei das im Verfahrensschritt c) zugeführte Volumen flüssiger Komponenten größer oder gleich 20% und kleiner oder gleich 200% bezogen auf die im Verfahrensschritt b) entnommene Katalysatorlösung beträgt.

5. Verfahren nach einem der vorhergehenden Ansprüche, wobei im Verfahrensschritt c) als Übergangsmetallquelle Rhodium-Verbindungen ausgewählt aus der Gruppe der Rhodium (III)-Salzen wie Rh-2-ethylhexanoat, -acetat, -oxalat, -propionat, - malonat, Rh(NO₃)₃, Rh(SO₄)₃, RhCl₃ oder der Rhodiumkomplexverbindungen wie Cyclopentadienylrhodiumverbindungen, [RhCl(Cyclooctadien-1,5)]₂, Rhodiumacetylacetonat, der Rhodiumcarbonylverbindungen wie Rh₃(CO)₁₂, Rh₆(CO)₁₆ oder in Form der verschiedenen Rhodiumoxide oder Mischungen mindestens zweier Verbindungen daraus zugegeben werden

6. Verfahren nach einem der Ansprüche 1-4, wobei im Verfahrensschritt c) als Übergangsmetallquelle präformierte Rhodium-Komplexe mit Organophosphorliganden zum Reaktionssystem zugegeben werden.

7. Verfahren nach einem der vorhergehenden Ansprüche, wobei im Verfahrensschritt c) Organophosphorliganden in einem molaren Verhältnis von größer oder gleich 20 und kleiner oder gleich 400 bezogen auf die in Verfahrensschritt b) entnommene Übergangsmetallmenge zugegeben werden.

8. Verfahren nach einem der vorhergehenden Ansprüche, wobei im Verfahrensschritt c) eine Lösung einer Rhodium-Verbindung umfassend Organophosphorliganden dem Reaktionssystem zugeführt wird.

9. Verfahren nach einem der Ansprüche 1-7, wobei im Verfahrensschritt c) vor der Zuführung der Übergangsmetallquelle Organophosphorliganden dem Reaktionssystem zugeführt werden.

10. Verfahren nach einem der Ansprüche 1-7, wobei im Verfahrensschritt c) zuerst eine Teilmenge des Lösemittels, dann die in einem Lösemittel gelöste Übergangsmetallquelle und anschließend die Organophosphorliganden dem Reaktionssystem zugeführt werden.

11. Verfahren nach einem der vorhergehenden Ansprüche, wobei durch die Verfahrensschritte b) und c) die Gesamtkonzentration vorliegender organischer Liganden und Salze in der wässrigen Lösung der Reaktionszone um größer oder gleich 10% und kleiner oder gleich 50% im Vergleich zu der Konzentration der organischen Liganden und der Salze in der wässrigen Lösung der Reaktionszone am Ende des Verfahrensschrittes a) reduziert werden.

12. Verfahren nach einem der vorhergehenden Ansprüche, wobei im Verfahrensschritt c) der pH-Wert in der wässrigen Lösung der Reaktionszone auf einen Bereich von größer oder gleich pH 4 und kleiner oder gleich pH 10 eingestellt wird.

13. Verfahren nach einem der vorhergehenden Ansprüche, wobei das molare Verhältnis von zugegebenen Organophosphorliganden zu zugegebenen Rhodium im Verfahrensschritt c) größer oder gleich 20 und kleiner oder gleich 500 beträgt.

14. Verfahren nach einem der vorhergehenden Ansprüche, wobei durch die Verfahrensschritte b) und c) die Konzentration der anorganischen Kationen in der Lösung der Reaktionszone um größer oder gleich 5% reduziert wird.

## Claims

1. A continuous two-phase hydroformylation process for the production of aldehydes from olefins by means of carbon monoxide, hydrogen and a transition metal catalyst in a reaction zone, the transition metal being in the form of a water-soluble catalyst complex, **characterised in that** the process comprises the following steps once or several times:
a) hydroformylating by reacting the olefins, carbon monoxide and hydrogen over a water-soluble transition metal catalyst comprising water-soluble organophosphorus ligands in the reaction zone;
b) reducing the concentration of the olefins in the reaction zone by reducing the olefin feed to the reaction zone by greater than or equal to 50% and less than or equal to 100% based on the concentration of olefin in the reaction zone in process step a) and withdrawing at least a portion of the catalyst solution from the reaction system, wherein the catalyst solution withdrawal and olefin concentration reduction sub-steps may occur in this or reverse order, simultaneously or sequentially;
c) feeding a solvent, a transition metal source and water-soluble organophosphorus ligands to the reaction system, wherein the feeding of the components may occur simultaneously or in any order sequentially;
d) increasing the concentration of the olefins in the reaction zone by increasing the olefin feed to the reaction zone and hydroformylating by reacting the olefins with carbon monoxide and hydrogen.

2. The process according to claim 1, wherein in process step b) the sub-step of reducing the olefin concentration occurs prior to the partial withdrawal of the catalyst solution.

3. The process according to any one of the preceding claims, wherein in process step b) a volume greater than or equal to 10% and less than or equal to 50% of aqueous catalyst solution is removed from the reaction system.

4. The process according to any one of the preceding claims, wherein the volume of liquid components added in process step c) is greater than or equal to 20% and less than or equal to 200% relative to the catalyst solution removed in process step b).

5. The process according to any one of the preceding claims, wherein in process step c), as transition metal source, rhodium compounds selected from the group of rhodium (III) salts such as Rh-2-ethylhexanoate, -acetate, -oxalate, -propionate, - malonate, Rh(NO₃)₃, Rh(SO₄)₃, RhCl₃or the rhodium complex compounds such as cyclopentadienylrhodium compounds, [RhCl(cyclooctadiene-1,5)]₂, rhodium acetylacetonate, the rhodium carbonyl compounds such as Rh₃(CO)₁₂, Rh₆(CO)₁₆ or in the form of various rhodium oxides or mixtures of at least two compounds thereof.

6. The process according to any one of claims 1-4, wherein in process step c) preformed rhodium complexes with organophosphorus ligands are added to the reaction system as a transition metal source.

7. The process according to any one of the preceding claims, wherein in process step c) organophosphorus ligands are added in a molar ratio of greater than or equal to 20 and less than or equal to 400 relative to the amount of transition metal removed in process step b).

8. The process according to any one of the preceding claims, wherein in process step c) a solution of a rhodium compound comprising organophosphorus ligands is fed to the reaction system.

9. The process according to any one of claims 1-7, wherein in process step c) organophosphorus ligands are added to the reaction system prior to addition of the transition metal source.

10. The process according to any one of claims 1-7, wherein in process step c), first a partial amount of the solvent, then the transition metal source dissolved in a solvent, and then the organophosphorus ligands are fed to the reaction system.

11. The process according to any one of the preceding claims, wherein by process steps b) and c) the total concentration of organic ligands and salts present in the aqueous solution of the reaction zone are reduced by greater than or equal to 10% and less than or equal to 50% compared to the concentration of organic ligands and salts in the aqueous solution of the reaction zone at the end of process step a).

12. The process according to any one of the preceding claims, wherein in process step c) the pH in the aqueous solution of the reaction zone is adjusted to a range of greater than or equal to pH 4 and less than or equal to pH 10.

13. The process according to any one of the preceding claims, wherein the molar ratio of added organophosphorus ligands to added rhodium in process step c) is greater than or equal to 20 and less than or equal to 500.

14. The process according to any one of the preceding claims, wherein by process steps b) and c) the concentration of inorganic cations in the reaction zone solution is reduced by greater than or equal to 5%.

## Revendications

1. Procédé d'hydroformylation continu à deux phases permettant la préparation d'aldéhydes à partir d'oléfines au moyen de mono oxyde de carbone, d'hydrogène et d'un catalyseur à métal de transition dans une zone réactionnelle, dans lequel le métal de transition est présent sous forme d'un complexe de catalyseur soluble dans l'eau, **caractérisé en ce que** le procédé comprend une fois ou plusieurs fois les étapes suivantes :
a) hydroformylation par la mise en réaction des oléfines, du mono oxyde de carbone et de l'hydrogène sur un catalyseur à métal de transition soluble dans l'eau présentant des ligands organo phosphorés dans une zone réactionnelle ;
b) réduction de la concentration des oléfines dans la zone réactionnelle par réduction de l'approvisionnement en oléfines vers la zone réactionnelle de plus ou de 50 % et de moins ou de 100 % par rapport à la concentration en oléfines dans la zone réactionnelle dans l'étape de procédé a) et retrait d'au moins une partie de la solution de catalyseur du système réactionnel, où les étapes partielles de retrait de la solution de catalyseur et de réduction de la concentration en oléfines peuvent avoir lieu dans cet ordre ou dans l'ordre inverse, simultanément ou l'une après l'autre ;
c) approvisionnement d'un solvant, d'une source de métal de transition et de ligands organo phosphorés dans le système réactionnel, où l'approvisionnement des composants peut avoir lieu simultanément ou dans un ordre quelconque l'un après l'autre ;
d) augmentation de la concentration des oléfines dans la zone réactionnelle par augmentation de l'approvisionnement en oléfines et hydroformylation en faisant réagir les oléfines avec le mono oxyde de carbone et l'hydrogène.

2. Procédé selon la revendication 1, dans lequel, dans l'étape de procédé b), l'étape partielle de la réduction de la concentration en oléfines a lieu avant le prélèvement partiel de la solution de catalyseur.

3. Procédé selon l'une des revendications précédentes, dans lequel, dans l'étape de procédé b), un volume égal ou supérieur à 10 % et inférieur ou égal à 50 % de solution aqueuse de catalyseur est prélevé du système réactionnel.

4. Procédé selon l'une des revendications précédentes, dans lequel le volume de composants liquides approvisionné dans l'étape de procédé c) est égal ou supérieur à 20 % et inférieur ou égal à 200 %, par rapport à la solution de catalyseur prélevée dans l'étape de procédé b).

5. Procédé selon l'une des revendications précédentes, dans lequel, dans l'étape de procédé c), en tant que source de métal de transition, des composés de rhodium choisis dans le groupe des sels de rhodium (III), comme le Rh-2-éthyl hexanoate, -acétate, -oxalate, -propionate, -malonate, Rh(NO₃)₃, Rh(SO₄)₃, RhCl₃ ou des composés complexes de rhodium comme des composés cyclo pentadiényl rhodium, le [RhCl(cyclo octadièn-1,5]₂, l'acétyl acétonate de rhodium, des composés carbonyl rhodium comme Rh₃(CO)₁₂, Rh₆(CO)₁₆ ou sous forme de divers oxydes de rhodium ou de mélanges d'au moins deux composés parmi ceux-ci sont ajoutés.

6. Procédé selon l'une des revendications 1 à 4, dans lequel, dans l'étape de procédé c), en tant que source de métal de transition, des complexes préformés avec des ligands organo phosphorés sont ajoutés au système réactionnel.

7. Procédé selon l'une des revendications précédentes, dans lequel, dans l'étape de procédé c), des ligands organo phosphorés sont ajoutés dans un rapport molaire égal ou supérieur à 20 et inférieur ou égal à 400, par rapport à la quantité de métal de transition prélevée dans l'étape de procédé b).

8. Procédé selon l'une des revendications précédentes, dans lequel, dans l'étape de procédé c), une solution d'un composé de rhodium comprenant des ligands organo phosphorés est ajoutée au système réactionnel.

9. Procédé selon l'une des revendications 1 à 7, dans lequel, dans l'étape de procédé c), des ligands organo phosphorés sont ajoutés au système réactionnel avant l'ajout de la source de métal de transition.

10. Procédé selon l'une des revendications 1 à 7, dans lequel, dans l'étape de procédé c), d'abord une partie du solvant, puis la source de métal de transition solubilisée dans un solvant et ensuite les ligands organo phosphorés sont ajoutés au système réactionnel.

11. Procédé selon l'une des revendications précédentes, dans lequel, par les étapes de procédé b) et c), la concentration totale de ligands organiques et de sels présents dans la solution aqueuse de la zone réactionnelle est réduite de plus ou de 10 % et de moins de ou de 50 %, comparativement à la concentration des ligands organiques et des sels dans la solution aqueuse de la zone réactionnelle à la fin de l'étape de procédé a).

12. Procédé selon l'une des revendications précédentes, dans lequel, dans l'étape de procédé c), la valeur du pH dans la solution aqueuse de la zone réactionnelle est réglée dans une plage égale ou supérieure à pH 4 et inférieure ou égale à pH 10.

13. Procédé selon l'une des revendications précédentes, dans lequel le rapport molaire de ligands organo phosphorés ajoutés sur le rhodium ajouté dans l'étape de procédé c) est égal ou supérieur à 20 et inférieur ou égal à 500.

14. Procédé selon l'une des revendications précédentes, dans lequel, par les étapes de procédé b) et c), la concentration des cations inorganiques dans la solution de la zone réactionnelle est réduite de plus de ou de 5 %.
